# EUROPÄISCHE PATENTSCHRIFT

(11) **EP 0 735 032 B1**
(45) Veröffentlichungstag und Bekanntmachung des Hinweises auf die Patenterteilung: **19.06.2002**
(21) Anmeldenummer: 96104387.4
(22) Anmeldetag: 20.03.1996
(51) Int. Cl.: C07D 307/60

(54) **Verfahren zur Herstellung von 5-Hydroxy-4-methyl-2(5H)-furanon**
Method for preparing 5-hydroxy-4-methyl-2(5H)-furanone
Procédé de préparation de 5-hydroxy-4-méthyl-2(5H)-furanon

(30) Priorität: 27.03.1995 DE 19510473
(43) Veröffentlichungstag der Anmeldung: 02.10.1996
(73) Patentinhaber: BASF AKTIENGESELLSCHAFT, 67056 Ludwigshafen (DE)
(72) Erfinder: John, Michael Dr., 67245 Lambsheim (DE); Dobler, Walter Dr., 69126 Heidelberg (DE); Paust, Joachim Dr., 67141 Neuhofen (DE)

(56) Entgegenhaltungen:
- JOURNAL OF THE CHEMICAL SOCIETY, PERKIN TRANSACTIONS 1, Nr. 6, 1981, LETCHWORTH GB, Seiten 1734-1743, XP002024940 A. W. JOHNSON ET AL.: "The Preparation of Synthetic Analogues of Strigol"
- DATABASE WPI Week 9435 Derwent Publications Ltd., London, GB; AN 94-283338 XP002024943 & JP 06 211 825 A (KURARAY CO LTD) , 2.August 1994
- JOURNAL OF ORGANIC CHEMISTRY, Bd. 44, Nr. 19, 1979, EASTON US, Seiten 3414-3416, XP002024941 G.K. COOPER ET AL.: "Convenient Synthesis of the 2-Methyl-4-hydroxybut-2-enolide Moiety of Strigol"
- JOURNAL OF ORGANIC CHEMISTRY, Bd. 46, Nr. 24, 1981, EASTON US, Seiten 4889-4894, XP002024942 J.J. BOURGUIGNON ET AL.: "Lactone Chemistry. Synthesis of beta-substituted, gamma-Functionalized Butaneloides and Butenolides and Succinaldehydic Acids from Glyoxylic Acid"

## Beschreibung

Die Erfindung betrifft ein auch technisch durchführbares Verfahren zur Herstellung von 5-Hydroxy-4-methyl-2(5H)-furanon (Butenolid) ausgehend von β-Formyl-crotonsäuremethyl- oder ethylester.

Butenolid ist ein wichtiger C₅-Baustein zur Herstellung von Retinoiden und Carotinoiden mit einer (Z)-konfigurierten Doppelbindung, wie beispielsweise 13(Z)-Retinsäure. Allein schon wegen des ständig wachsenden Bedarfs an 13(Z)-Retinsäure zur Bekämpfung von Akne oder frühzeitiger Alterung der Haut hat es nicht an Versuchen gefehlt, ein vorteilhaftes auch technisch gut durchführbares Verfahren zur Herstellung von Butenolid zu finden.

So ist aus J. Org. Chem., 46, (1981), Seiten 4889-94, bekannt, daß man durch Mannich-Reaktion von Succindialdehyd mit Morpholin und Glyoxylsäure in basischem oder neutralem Medium α,γ-Dimorpholino-butanolid und α-Morpholino-γ-hydroxy-butanolid herstellen kann. Aus letzterem läßt sich in saurem Medium die α-Morpholinogruppe abspalten, wodurch Butenolid gebildet wird. Nachteilig an diesem Verfahren ist, daß das Verfahren mehrere Reaktionsstufen beinhaltet und größere Mengen an Morpholin als zusätzlichem Einsatzstoff notwendig sind.

Weiterhin war aus J. Org. Chem. Vol. 44, No. 19 (1979), Seiten 3414-16, ein Verfahren zur Herstellung von 5-Hydroxy-3-methyl-2(5H)-furanon (einem Isomeren von Butenolid) bekannt, bei dem Crotonaldehyd in fünf sehr aufwendigen Reaktionsstufen in β-Formyl-methacrylsäureethylester überführt wird und letzterer durch Erhitzen mit Schwefelsäure in 72 %iger Ausbeute in 5-Hydroxy-3-methyl-2(5H)-furanon überführt wird.

Nachteilig auch an diesem Verfahren ist, daß das Butenolid-Isomere in sechs teilweise sehr aufwendigen Reaktionsstufen und unter Mitverwendung von teuren Einsatzstoffen, wie n-Butyllithium und Chlorameisensäureethylester hergestellt werden muß.

Es war weiterhin aus dem Derwent-Referat von JP-A 62 11825 bekannt, daß man 3-Methyl-4-oxo-2-hydroxy-butansäureester unter salzsauren Bedingungen zu 5-Hydroxy-4-methyl-2(5H)-furanon cyclisieren kann. Nachteilig an diesem Verfahren ist, daß man pro Mol Edukt etwa 4 Mol Salzsäure benötigt.

Es war daher die Aufgabe der Erfindung, ein Verfahren zur Herstellung von Butenolid zu entwickeln, welches es erlaubt, Butenolid ausgehend von leicht zugänglichen Ausgangsstoffen auf einfache Weise und mit guten Ausbeuten so vorteilhaft herzustellen, daß auch eine vorteilhafte Herstellung in technischem Maßstab möglich wird.

Es wurde nun überraschenderweise gefunden, daß sich β-Formyl-crotonsäurealkylester unter speziellen Bedingungen sehr vorteilhaft durch Erhitzen mit wäßriger Salzsäure in das Butenolid überführen lassen. Dieses Ergebnis war überraschend, da die Cyclisierung des β-Formyl-crotonsäureesters mit verdünnter Schwefelsäure gemäß dem Verfahren in J. Org. Chem. Vol. 44, No. 19 (1979), Seiten 3414-16, nur zu einem Gemisch aus dem Edukt, dem Butenolid, dem 5-Methoxy-4-methyl-2(5H)-furanon und einer Vielzahl von Zersetzungsprodukten führt (siehe Vergleichsbeispiele 3a und 3b).

Da β-Formyl-crotonsäuremethylester durch Aldolkondensation von Propanal mit Glyoxylsäuremethylester auf einfache Weise und in guten Ausbeuten zugänglich ist, ergibt sich somit ein vorteilhaftes Gesamtverfahren ausgehend von kommerziell erhältlichen billigen Ausgangsstoffen.

Gegenstand der Erfindung ist daher ein Verfahren zur Herstellung von 5-Hydroxy-4-methyl-2(5H)-furanon der Formel I 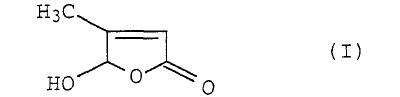 das dadurch gekennzeichnet ist, daß man einen β-Formyl-crotonsäurealkylester der allgemeinen Formel II 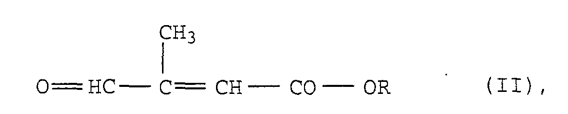 in der R für eine Methyl- oder Ethylgruppe, vorzugsweise eine Methylgruppe steht, in Gegenwart von 0,01 bis 1 Mol Methanol oder Ethanol pro Mol β-Formyl-crotonsäurealkylester mit 0,1 bis 2 Mol, vorzugsweise 0,6 bis 1,2 Mol, insbesondere 0,8 bis 1 Mol pro Mol β-Formyl-crotonsäurealkylester an Salzsäure in Form einer 1 bis 38 gew.-%igen wäßrigen Salzsäure für 0,5 bis 24 Stunden, vorzugsweise 0,5 bis 4 Stunden auf Temperaturen von 90 bis 110°C erhitzt.

Die Tatsache, daß durch Zusatz einer katalytischen Menge an Methanol bzw. Ethanol als Lösungsvermittler zum Reaktionsgemisch die Ausbeute an Butenolid bei dieser Umsetzung beträchtlich gesteigert werden kann, war überraschend, da der Zusatz von höheren Alkanolen, wie dem Isopropanol oder Isobutanol, die Ausbeute an dem gewünschten Butenolid nicht steigerte, sondern zur Bildung von Vollacetalen führte.

Mit Vorteil verwendet man für die Umsetzung des β-Formyl-crotonsäuremethylester Methanol und für die Umsetzung des Ethylesters Ethanol, da anderenfalls teilweise Umesterung erfolgt und dadurch ein uneinheitliches Produkt gebildet wird.

Erfindungsgemäß verwendet man das Methanol bzw. das Ethanol in Mengen von 0,01 bis 1 Mol, vorzugsweise 0,01 bis 0,6 Mol, insbesondere 0,01 bis 0,1 Mol pro Mol β-Formyl-crotonsäurealkylester.

Im erfindungsgemäßen Verfahren kann man als Ausgangsverbindung den β-Formyl-crotonsäurealkylester in Form eines Gemisches aus seinem E- und Z-Isomeren, wie es bei der Aldolkondensation von Propanal mit Glyoxylsäurealkylester anfällt, einsetzen, da das hierin enthaltende E-Isomere unter den Reaktionsbedingungen in das cyclisierbare Z-Isomere isomerisiert.

Zur Durchführung des erfindungsgemäßen Verfahrens geht man beispielsweise mit Vorteil so vor, daß man β-Formyl-crotonsäuremethylester mit der wäßrigen Salzsäure und gegebenenfalls mit Methanol versetzt und das Reaktionsgemisch für die Reaktionszeit unter Rückfluß zum Sieden erhitzt, d.h. etwa auf 98°C erhitzt. Die zunächst gebildete Emulsion löst sich dabei innerhalb von etwa 15 Minuten nach Erreichen der Rückflußbedingungen auf.

Die Reaktionszeit beträgt im allgemeinen 0,5 bis 24 Stunden, vorzugsweise 0,5 bis 4, insbesondere 0,5 bis 3 Stunden.

Zur Aufarbeitung kann man aus dem Reaktionsgemisch unter vermindertem Druck Wasser, Methanol und Salzsäure abdestillieren und dann das Butenolid aus dem überwiegend aus dem gewünschten Butenolid und 5-Methoxy-4-methyl-2(5H)-furanon als Nebenprodukt bestehenden Reaktionsgemisch durch Destillation gewinnen.

Auch das als Nebenprodukt gebildete 5-Methoxy-4-methyl-2(5H)-furanon kann durch Erhitzen mit etwa 0,1 bis 1,2 Mol, vorzugsweise etwa 1 Mol verdünnter Salzsäure pro Mol der 5-Methoxy-Verbindung auf Temperaturen von 90 bis 110°C, vorzugsweise 95 bis 100°C zu etwa 90 % in das gewünschte Butenolid überführt werden, wodurch mit Hilfe des erfindungsgemäßen Verfahrens insgesamt eine nahezu vollständige Umsetzung des eingesetzten β-Formyl-crotonsäureesters möglich ist.

Als verdünnte Salzsäure verwendet man auch hierbei eine 1 bis 38 gew.-%ige, vorzugsweise eine 2 bis 20 gew.-%ige, insbesondere eine etwa 3 bis 10 gew.-%ige wäßrige Salzsäure.

Besonders vorteilhaft gestaltet sich das erfindungsgemäße Verfah- . ren jedoch, wenn man aus dem Reaktionsgemisch nach der Cyclisierung unter vermindertem Druck im wesentlichen nur Methanol, Wasser und einen Teil der Salzsäure abdestilliert und das noch salzsaure Rohprodukt anschließend 0,25 bis 8 Stunden, vorzugsweise 0,3 bis 6 Stunden, auf Temperaturen von 90 bis 110°C erhitzt, wobei das im Reaktionsgemisch enthaltene 5-Alkoxy-4-methyl-2(5H)-furanon in das gewünschte Butenolid übergeführt wird.

Ganz besonders vorteilhaft gestaltet sich das erfindungsgemäße Verfahren, wenn man β-Formyl-crotonsäuremethylester in Gegenwart von 0,01 bis 0,6 Mol Methanol und etwa 0,8 bis 1,2 Mol Salzsäure in Form einer etwa 2 bis 20 gew.-%igen wäßrigen Salzsäure pro Mol β-Formyl-crotonsäuremethylester für 0,5 bis 4 Stunden unter Rückfluß zum Sieden erhitzt, anschließend unter vermindertem Druck im wesentlichen Methanol und Wasser neben einem Teil der Salzsäure abdestilliert und das noch salzsäurehaltige Rohprodukt noch etwa 2 Stunden unter letztlich bis auf 0,6 mbar vermindertem Druck auf Temperaturen von 90 bis 110°C erhitzt. Praktisch bedeutet dies, daß man das noch salzsäurehaltige Rohprodukt unter einem beispielsweise durch eine Dampfstrahlvakuumpumpe verminderten Druck so lange auf 90 bis 110°C erhitzt, bis sich ein Druck von etwa 0,6 mbar eingestellt hat.

Durch destillative Aufarbeitung erhält man das Butenolid auf diese Weise direkt in einer Eintopfreaktion in einer Ausbeute von bis zu 96 % der Theorie.

Mit Hilfe des erfindungsgemäßen Verfahrens kann das zur Herstellung von Retinoiden und Carotinoiden mit (Z)-Konfiguration begehrte Butenolid auch technisch auf einfache Weise und in sehr guten Ausbeuten aus gut zugänglichen Ausgangsstoffen hergestellt werden.

### Beispiel 1

### Cyclisierung in Anwesenheit von Methanol

409,2 g (3,124 mol) β-Formyl-crotonsäuremethylester wurden mit 10 g (0,312 mol) Methanol und 1366,8 g (1,874 mol) einer 5 %igen HCl versetzt und 2 Stunden (h) unter Rückfluß auf 98°C erhitzt. Die zunächst vorliegende Emulsion löste sich innerhalb von 15 Minuten nach Beginn der Rückflußbedingungen auf. Eine gaschromatographische Analyse (GC-Analyse) des erhaltenen Rohprodukts zeigte einen Anteil von 12 % 5-Methoxy-4-methyl-2(5H)-furanon und 87,1 % Butenolid. Das derart zusammengesetzte Reaktionsprodukt wurde bei 90°C und 10 mbar unter Entfernung von Wasser, Methanol und HCl eingeengt, bevor bei 1 mbar eine fraktionierte Destillation zur Abtrennung des als Nebenprodukt auftretenden 5-Methoxy-4-methyl-2(5H)-furanons erfolgte.

Eine Destillation bei 1 mbar Druck ergab 6,75 g einer Fraktion 1, 328,9 g einer Fraktion 2 und 9,72 g Rückstand.

Mittels GC-Analyse (25 m OV1701, 50/10/240) wurde folgende Zusammensetzung der Fraktionen ermittelt:

| | |
|---|---|
| Fraktion 1 | 34,9 % 5-Methoxy-4-methyl-2(5H)-furanon 63,8 % Butenolid |
| Fraktion 2 | 97 % Butenolid |

Bezogen auf die isolierte Menge Butenolid (Fraktion 2) wurde somit eine Ausbeute von 92 der Theorie erreicht.

Zur Verbesserung der Gesamtausbeute wurde das als Nebenprodukt auftretende 5-Methoxy-4-methyl-2(5H)-furanon in das gewünschte Butenolid überführt. Dazu wurden 81,86 g (0,64 mol) 5-Methoxy-4-methyl-2(5H)-furanon mit 465,9 g (0,64 mol) einer 5 gew.-%igen HCl für 2,5 h unter Rückfluß zum Sieden erhitzt. Die GC-Analyse des Reaktionsproduktes ergab eine Zusammensetzung von 10,1 % Edukt und 89,1 % Butenolid, so daß insgesamt eine nahezu vollständige Umsetzung des ursprünglich eingesetzten β-Formyl-crotonsäuremethylesters möglich ist.

### Beispiel 2 (nicht erfindungsgemäß)

### Cyclisierung ohne Anwesenheit von Methanol

102,3 g β-Formyl-crotonsäuremethylester und 38,4 g einer 38 %igen wäßrigen Salzsäure wurden zusammen für 3 h unter Rückfluß zum Sieden erhitzt.

Eine GC-Analyse des erhaltenen Rohproduktes zeigte einen Anteil von 53 % 5-Methoxy-4-methyl-2(5H)-furanon und 42 % an dem gewünschten Butenolid.

Das so erhaltene Rohprodukt wurde bei 10 mbar unter Entfernung von Wasser und Salzsäure eingeengt und dann fraktioniert destilliert.

Das erhaltene 5-Methoxy-4-methyl-2(5H)-furanon wurde analog Beispiel 1 durch Erhitzen mit einer 5 gew.-%igen wäßrigen Salzsäure in das gewünschte Butenolid überführt.

Die Gesamtausbeute an Butenolid betrug 81 % der Theorie.

### Beispiel 3 (Vergleichsbeispiele)

a) Ohne Zusatz von Methanol
   102,3 g β-Formyl-crotonsäuremethylester wurden zusammen mit 200 ml einer 4n H₂SO₄ für 3 h unter Rückfluß zum Sieden erhitzt. Eine GC-Analyse des erhaltenen Rohproduktes ergab folgende Zusammensetzung:
   25 % unumgesetztes Edukt,
   18 % Butenolid,
   19 % 5-Methoxy-4-methyl-2(5H)-furanon und
   38 % einer Vielzahl von Zersetzungsprodukten.
b) Unter Zusatz von Methanol
   Man arbeitete wie in Beispiel 3a, jedoch unter Zusatz von 32 ml Methanol. Eine GC-Analyse des erhaltenen Rohproduktes ergab folgende Zusammensetzung:
   43 % unumgesetztes Edukt,
   9 % Butenolid,
   22 % 5-Methoxy-4-methyl-2(5H)-furanon und
   26 % einer Vielzahl von Zersetzungsprodukten.

### Beispiel 4

512 g (3,9 mol) β-Formyl-crotonsäuremethylester wurden bei Raumtemperatur mit 12,5 g (0,39 mol) Methanol und 1,4 kg einer 10 %igen wäßrigen Salzsäure versetzt, das Gemisch unter Rühren und Rückflußkühlung für 2 h zum Sieden erhitzt und danach auf Raumtemperatur abgekühlt. Anschließend wurden unter vermindertem Druck (10 bis 20 mbar) bei 90°C die enthaltenen Leichtsieder abdestilliert und das noch salzsäurehaltige Reaktionsgemisch unter einem durch eine Dampfstrahlvakuumpumpe verminderten Druck auf ca. 90°C erhitzt bis sich ein Druck von etwa 0,6 mbar einstellte, wozu etwa 2 h notwendig waren.

Mittels GC-Analyse wurde festgestellt, daß das Butenolid in dieser Eintopfreaktion in einer Ausbeute von 96 % der Theorie und in einer Reinheit von 96 bis 97 % erhalten wurde.

### Beispiel 5

Analog Beispiel 4 wurden 566,6 g (3,91 mol) β-Formyl-crotonsäureethylester mit 17,9 g (0,39 mol) Ethanol und 1,4 kg einer 10 %igen wäßrigen Salzsäure versetzt, das Gemisch unter Rühren und Rückflußkühlung für 2 h zum Sieden erhitzt und dann auf Raumtemperatur abgekühlt. Durch Abdestillieren der Leichtsieder und 2-stündiges Erhitzen des Reaktionsgemisches analog Beispiel 4 unter vermindertem Druck auf 90°C wurde Butenolid in einer Ausbeute von 93 % der Theorie in einer Reinhiet von 96 bis 98 % erhalten.

## Patentansprüche

1. Verfahren zur Herstellung von 5-Hydroxy-4-methyl-2(5H)-furanon der Formel I 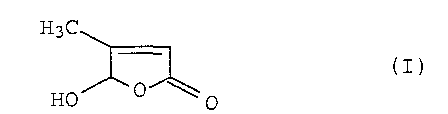 **dadurch gekennzeichnet, daß** man einen β-Formyl-crotonsäurealkylester der allgemeinen Formel II 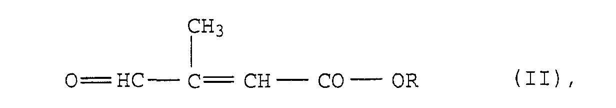 in der R für eine Methyl- oder Ethylgruppe steht, in Gegenwart von 0,01 bis 1 Mol Methanol oder Ethanol pro Mol β-Formyl-crotonsäurealkylester mit 0,1 bis 2 Mol pro Mol β-Formyl-crotonsäureester an Salzsäure in Form einer 1 bis 38 gew.-%igen wäßrigen Salzsäure für 0,5 bis 24 Stunden auf Temperaturen von 90 bis 110°C erhitzt.

2. Verfahren gemäß Anspruch 1, **dadurch gekennzeichnet, daß** man β-Formyl-crotonsäuremethylester als β-Formyl-crotonsäurealkylester einsetzt.

3. Verfahren gemäß Anspruch 1, **dadurch gekennzeichnet, daß** man den β-Formyl-crotonsäureester der Formel II mit 0,6 bis 1,2 Mol wäßriger Salzsäure erhitzt.

4. Verfahren gemäß Anspruch 1, **dadurch gekennzeichnet, daß** man den β-Formyl-crotonsäureester der Formel II mit einer 2 bis 20 gew.-%igen wäßrigen Salzsäure erhitzt.

5. Verfahren gemäß Anspruch 1, **dadurch gekennzeichnet, daß** man den β-Formyl-crotonsäurealkylester der Formel II in Gegenwart von 0,01 bis 1 Mol Methanol pro Mol β-Formyl-crotonsäureester mit der wäßrigen Salzsäure erhitzt.

6. Verfahren gemäß Anspruch 1, **dadurch gekennzeichnet, daß** man den β-Formyl-crotonsäurealkylester in Gegenwart von 0,01 bis 0,6 Mol Methanol pro Mol β-Formyl-crotonsäureester mit der wäßrigen Salzsäure erhitzt.

7. Verfahren gemäß Anspruch 2, **dadurch gekennzeichnet, daß** man den β-Formyl-crotonsäuremethylester in Gegenwart von 0,01 bis 0,6 Mol Methanol und etwa 0,8 bis 1,2 Mol Salzsäure in Form einer etwa 2 bis 20 gew.-%igen wäßrigen Salzsäure pro Mol β-Formyl-crotonsäuremethylester für 0,5 bis 4 Stunden unter Rückfluß zum Sieden erhitzt.

8. Verfahren gemäß Anspruch 1, **dadurch gekennzeichnet, daß** man das bei der Umsetzung als Nebenprodukt gebildete 5-Alkoxy-4-methyl-2(5H)-furanon isoliert und durch Erhitzen mit verdünnter Salzsäure in das 5-Hydroxy-4-methyl-2(5H)-furanon der Formel I überführt.

9. Verfahren gemäß Anspruch 2, **dadurch gekennzeichnet, daß** man den β-Formyl-crotonsäuremethylester in Gegenwart von 0,01 bis 0,6 Mol Methanol und etwa 0,8 bis 1,2 Mol Salzsäure in Form einer etwa 2 bis 20 gew.-%igen wäßrigen Salzsäure pro Mol β-Formyl-crotonsäuremethylester für 0,5 bis 4 Stunden unter Rückfluß zum Sieden erhitzt, anschließend unter vermindertem Druck im wesentlichen Methanol und Wasser abdestilliert, das noch salzsäurehaltige Rohprodukt noch 0,25 bis 6 Stunden auf Temperaturen von 90 bis 110°C erhitzt und das so behandelte Reaktionsgemisch fraktioniert destilliert.

10. Verfahren gemäß Anspruch 2, **dadurch gekennzeichnet, daß** man den β-Formyl-crotonsäuremethylester in Gegenwart von 0,01 bis 0,6 Mol Methanol und etwa 0,8 bis 1,2 Mol Salzsäure in Form einer etwa 2 bis 20 gew.-%igen wäßrigen Salzsäure pro Mol β-Formyl-crotonsäuremethylester für 0,5 bis 4 Stunden unter Rückfluß zum Sieden erhitzt, anschließend unter vermindertem Druck im wesentlichen Methanol und Wasser abdestilliert, das noch salzsäurehaltige Rohprodukt noch etwa 2 Stunden unter einem letztlich bis auf 0,6 mbar verminderten Druck auf Temperaturen von 90 bis 110°C erhitzt und das so behandelte Reaktionsgemisch fraktioniert destilliert.

## Claims

1. A process for preparing 5-hydroxy-4-methyl-2(5H)-furanone of the formula I 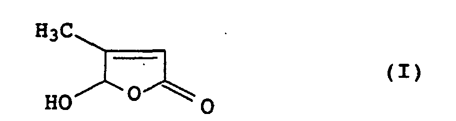 which comprises treating an alkyl β-formylcrotonate of the formula II 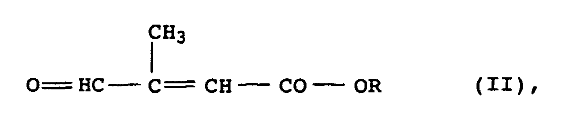 where R is methyl or ethyl, in the presence of from 0.01 to 1 mol of methanol or ethanol per mol of alkyl β-formylerotonate with from 0.1 to 2 mol of hydrochloric acid, in the form of 1-38% by weight aqueous hydrochloric acid, per mol of β-formylcrotonate at from 90 to 110°C for 0.5-24 hours.

2. A process as claimed in claim 1, wherein methyl β-formylerotonate is used as alkyl β-formylerotonate.

3. A process as claimed in claim 1, wherein the β-formylerotonate of the formula II is heated with from 0.6 to 1.2 mol of aqueous hydrochloric acid.

4. A process as claimed in claim 1, wherein the β-formylcrotonate of the formula II is heated with 2-20% by weight aqueous hydrochloric acid.

5. A process as claimed in claim 1, wherein the alkyl β-formylcrotonate of the formula II is heated with the aqueous hydrochloric acid in the presence of from 0.01 to 1 mol of methanol per mol of β-formylcrotonate.

6. A process as claimed in claim 1, wherein the alkyl β-formylcrotonate is heated with the aqueous hydrochloric acid in the presence of from 0.01 to 0.6 mol of methanol per mol of β-formylcrotonate.

7. A process as claimed in claim 2, wherein the methyl β-formylcrotonate is refluxed in the presence of from 0.01 to 0.6 mol of methanol and about 0.8-1.2 mol of hydrochloric acid in the form of approximately 2-20% by weight aqueous hydrochloric acid per mol of methyl β-formylcrotonate for 0.5-4 hours.

8. A process as claimed in claim 1, wherein the 5-alkoxy-4-methyl-2(5H)-furanone formed as byproduct in the reaction is isolated and converted by heating with dilute hydrochloric acid into 5-hydroxy-4-methyl-2(5H)-furanone of the formula I.

9. A process as claimed in claim 2, wherein the methyl β-formylcrotonate is refluxed in the presence of from 0.01 to 0.6 mol of methanol and about 0.8-1.2 mol of hydrochloric acid in the form of approximately 2-20% by weight aqueous hydrochloric acid per mol of methyl β-formylcrotonate for 0.5-4 hours, subsequently essentially methanol and water are removed by distillation under reduced pressure, the crude product, which still contains hydrochloric acid, is heated at from 90 to 110°C for 0.25-6 hours, and the reaction mixture treated in this way is fractionally distilled.

10. A process as claimed in claim 2, wherein the methyl β-formylcrotonate is refluxed in the presence of from 0.01 to 0.6 mol of methanol and about 0.8-1.2 mol of hydrochloric acid in the form of approximately 2-20% by weight aqueous hydrochloric acid per mol of methyl β-formylcrotonate for 0.5-4 hours, subsequently essentially methanol and water are removed by distillation under reduced pressure, the crude product, which still contains hydrochloric acid, is heated at from 90 to 110°C under a pressure which is finally reduced to 0.6 mbar for about 2 hours, and the reaction mixture treated in this way is fractionally distilled.

## Revendications

1. Procédé pour la préparation de la 5-hydroxy-4-méthyl-2(5H)-furannone de formule I 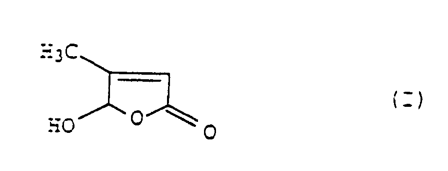 **caractérisé par le fait que** l'on chauffe un β-formylcrotonate d'alkyle de formule générale II 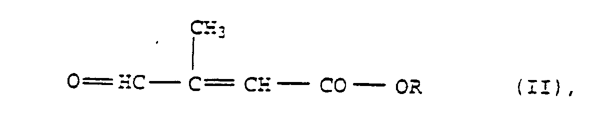 dans laquelle R représente un groupe méthyle ou éthyle, pendant 0,5 à 24 h à des températures de 90 à 110°C, en présence de 0,01 à 1 mol de méthanol ou d'éthanol par mol du β-formylcrotonate d'alkyle, avec 0,1 à 2 mol d'acide chlorhydrique à l'état d'acide chlorhydrique aqueux à une concentration de 1 à 38 % en poids, par mol de l'ester β-formylcrotonique.

2. Procédé selon la revendication 1, **caractérisé par le fait que** le β-formylcrotonate d'alkyle mis en oeuvre est le β-formylcrotonate de méthyle.

3. Procédé selon la revendication 1, **caractérisé par le fait que** l'on chauffe l'ester β-formylcrotonique de formule II avec 0,6 à 1,2 mol d'acide chlorhydrique aqueux.

4. Procédé selon la revendication 1, **caractérisé par le fait que** l'on chauffe l'ester β-formylcrotonique de formule II avec un acide chlorhydrique aqueux à une concentration de 2à 20 % en poids.

5. Procédé selon la revendication 1, **caractérisé par le fait que** l'on chauffe le β-formylcrotonate d'alkyle de formule II avec l'acide chlorhydrique aqueux en présence de 0,01 à 1 mol de méthanol par mol de l'ester β-formylcrotonique.

6. Procédé selon la revendication 1, **caractérisé par le fait que** l'on chauffe le β-formylcrotonate d'alkyle avec l'acide chlorhydrique aqueux en présence de 0,01 à 0,6 mol de méthanol par mol de l'ester β-formylcrotonique.

7. Procédé selon la revendication 2, **caractérisé par le fait que** l'on chauffe le β-formylcrotonate de méthyle à l'ébullition au reflux pendant 0,5 à 4 h en présence de 0,01 à 0,6 mol de méthanol et environ 0,8 à 1,2 mol d'acide chlorhydrique à l'état d'acide chlorhydrique aqueux à une concentration d'environ 2 à 20 % en poids par mol du β-formylcrotonate de méthyle.

8. Procédé selon la revendication 1, **caractérisé par le fait que** l'on isole la 5-alcoxy-4-méthyl-2(5H)-furannone formée en produit d'accompagnement dans la réaction et on la convertit par chauffage avec de l'acide chlorhydrique dilué en la 5-hydroxy-4-méthyl-2(5H)-furannone de formule I.

9. Procédé selon la revendication 2, **caractérisé par le fait que** l'on chauffe le β-formylcrotonate de méthyle à l'ébullition au reflux pendant 0,5 à 4 h en présence de 0,01 à 0,6 mol de méthanol et d'environ 0,8 à 1,2 mol d'acide chlorhydrique à l'état d'acide chlorhydrique aqueux à une concentration d'environ 2 à 20 % en poids par mol du β-formylcrotonate de méthyle, on distille ensuite sous vide essentiellement le méthanol et l'eau, on chauffe le produit brut contenant encore de l'acide chlorhydrique pendant 0,25 à 6 h à des températures de 90 à 110°C et on soumet le mélange de réaction ainsi traité à une distillation fractionnée.

10. Procédé selon la revendication 2, **caractérisé par le fait que** l'on chauffe le β-formylorotonate de méthyle à l'ébullition au reflux pendant 0,5 à 4 h en présence de 0,01 à 0,6 mol de méthanol et d'environ 0,8 à 1,2 mol d'acide chlorhydrique à l'état d'acide chlorhydrique aqueux à une concentration d'environ 2 à 20 % en poids, par mol du β-formylcrotonate de méthyle, on distille ensuite sous vide essentiellement le méthanol et l'eau, on chauffe le produit brut contenant encore de l'acide chlorhydrique pendant 2 h environ sous un vide tombant finalement à 0,6 mbar à des températures de 90 à 110°C et on soumet le mélange de réaction ainsi traité à distillation fractionnée.
